# EUROPEAN PATENT SPECIFICATION

(11) **EP 1 560 808 B1**
(45) Date of publication and mention of the grant of the patent: **20.05.2009**
(21) Application number: 03799464.7
(22) Date of filing: 13.11.2003
(51) Int. Cl.: C07C 253/34

(54) **PURIFICATION/DECOLORIZATION TREATMENT FOR FATTY NITRILES**
BEHANDLUNG ZUR REINIGUNG/ENTFÄRBUNG VON FETTSÄURENITRILEN
TRAITEMENT DE PURIFICATION/DECOLORATION DE NITRILES GRAS

(30) Priority: 15.11.2002 US 426537 P
(43) Date of publication of application: 10.08.2005
(73) Proprietor: Akzo Nobel N.V., 6824 BM Arnhem (NL)
(72) Inventor: TELSCHOW, Jeffrey, Earl, Croton-on Hudson, NY 10520 (US)
(74) Representative: Schalkwijk, Pieter Cornelis
(86) International application number: PCT/EP2003/012834
(87) International publication number: WO 2004/046067

(56) References cited:
- GB-A- 526 496
- US-A- 4 575 434
- US-A1- 2002 120 005

## Description

### Field of the Invention

The present invention generally relates to a new purification and/or decolorization treatment process for fatty nitriles.

### Background of the Invention

Fatty nitriles, those derived from fatty acids, are important articles of commerce which are useful in the preparation of insecticides, gelling agents, fabric softeners and wetting agents. They may be hydrogenated to form primary, secondary or tertiary amines, particularly the valuable aliphatic amines.

A common commercial method for obtaining nitriles is the catalytic dehydration of fatty acids in the presence of ammonia. This method, however, yields, in addition to the nitriles, many troublesome by-products, including different amines that contribute color and odor, and also amides. Such impurities, particularly the amides, must be reduced to a low level before the nitriles may be used in the various processes that produce the valuable products. Amides are known to be poisons for hydrogenation catalysts.

There are many teachings in the prior art that deal with the purification of nitriles, usually by removing those by-products of the nitrile synthesis reaction, primarily color bodies and amines, which are close in boiling point to the nitrile product and, therefore, difficult to remove by distillation. In the process of U.S. Patent No. 2,622,097 to Osborne, impure acrylonitrile is first passed through activated carbon then through a moistened ion exchange material which may be regenerated by the use of acid, but which is substantially free of acid when used.

The process of U.S. Patent No. 2,943,049 removes hydrocarbon compounds of nitrogen, particularly amines, from hydrocarbon mixtures using various siliceous minerals, including bentonite which have been completely hydrogen ion exchanged.

U.S. Patent No. 3,206,497 to Oblad discloses the separation of a nitrile from a mixture containing other nitrogen-containing compounds of basic character by contacting the mixture with a metal halide which precipitates out the basic nitrogen compound.

In the process of U.S. Patent No. 3,262,966 to Higgins, Jr. et al, activated alumina is used to remove carbonyl compound impurities from acrylonitrile.

In the process of U.S. Patent No. 4,147,717 to Kershaw, amines are removed from adiponitrile by using a variety of materials, including bentonite in the presence of acid, with the adiponitrile preferably containing from 1 to 10% by weight of water.

The process of Great Britain Patent No. 1,223,790 to Kuhlmann effects removal of impurities from nitriles, particularly heterocyclic compounds containing one or more nitrogen atoms in the ring, by using various adsorbents, including montmorillonite.

German Pat. No. 1,046,601 to Cadus et al discloses a process for the purification of adiponitrile using a solid adsorbent such as silica gel, activated carbon or clay.

U.S. Patent No. 4,575,434 discloses a process for the removal of long-chain aliphatic amides from a solution of said amides and fatty acid derived nitriles. Here, both a layered mineral comprising an aluminium silicate and an acid strong enough to protonate said amides is employed.

It is also known to the art that organic cations, particulary amines, may replace cations which were originally present on clay surfaces and that there is a strong preference of the clay for the organic cation. Clay Colloid Chemistry, Van Olphen, H., Wiley, 2nd Ed., 1977 is one reference that provides such a teaching.

The present invention addresses a problem not specifically addressed by any of the above references comprising the presence of amides as an impurity in the nitriles. Nitriles produced via the above reaction in which fatty acids are dehydrated in the presence of catalyst and ammonia may contain amides in solution up to the saturation point (about 0.9 wt. % amide). The prior art method for removing these amides has been simple distillation, which is sometimes feasible because of the significant difference in boiling points between the nitriles and amides. If one, however, has to work with nitriles of mixed chain lengths, distillative separations may not always be possible due to the fact that amides of shorter chain length codistill with the higher chain length nitriles. Distillation, also, requires considerable energy input which in an era of increasing energy costs becomes increasingly unattractive.

The present inventors have discovered a process that effects removal of amides from solution with nitriles without employing distillation.

### Summary of the Invention

The primary objective to which the present invention is directed is the removal of amides from a solution of nitriles and amides. In one embodiment, the invention contemplates a process for the removal of impurities comprising amides, but which may include other impurities such as amines, from a solution of the nitriles and impurities. A reaction mixture is first formed and to that reaction mixture, 0.5 to 3wt% of diluted H₂SO₄ (approximately 60%) is stirred in at room temperature and atmospheric pressure for a time effective for the removal of the amide impurities. Typically, after about 30 minutes, the amide impurity is removed into the lower, dark acid layer, probably as a salt. The remaining nitrile, after decantation or filtration, is still colored but is nearly free of amide. In a second step, at room temperature or at a temperature of up to ∼80°C, color can be removed from the nitrile by stirring the acid-washed nitrile with 0.5-5 wt% of an adsorbent such as clay (bentonite), charcoal, zeolitic type materials, mixtures thereof and the like.

### Detailed Description of the Invention

Nitrile feedstocks of primary interest to the present invention fall into one of the three types comprising "coco-nitrile", "tallow-nitrile" and "oleo-nitrile". The compositions of these types, in terms of percent of fatty nitriles of various chain lengths, are in accordance with the following:

| No. of Carbons | 6 | 8 | 10 | 12 | 14 | 16 | 18 | 14' | 16' | 18' | 18" |
|---|---|---|---|---|---|---|---|---|---|---|---|
| Coco-nitrile | .5 | 8 | 7 | 50 | 18 | 8 | 1.5 | | | 6 | 1 |
| Tallow-nitrile | | | | 1 | 3 | 29 | 23 | 1 | 3 | 37 | 1.5 |
| Oleo-nitrile | | | | .5 | 3.5 | 4 | 5 | 1.5 | 5 | 76 | 3 |

The superscripts ' and " denote, one unsaturated and two unsaturated bonds respectively, per molecule.

The amide impurity with which the present invention is particularly concerned has the chemical structure: 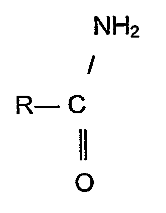 where R may comprise a wide variety of hydrocarbon or hydrocarbon-based groups, particularly
long chain aliphatics. The term "long chain" denominating compounds with, on average, 6 or more, preferably 8 or more, and most preferably 10 or more carbon atoms. The reaction mixture of the invention will include such amides. The amide content in the nitrile may be as high as its solubility limit (up to about 0.9 wt. % at room temperature) or even greater, whereupon at least a portion of the amides would appear as particulate matter. The average molecular weight of the amides in the above feedstock is assumed hereinafter to be 270. Amides of that molecular weight are referred to as "tallow amides". There may also be a wide variety of other impurities, primarily amines, which may impart color and odor to the nitriles.

The present invention is directed is the removal of amides from a solution of nitriles and amides. In one embodiment, the invention contemplates a process for the removal of impurities comprising amides, but which may include other impurities such as amines, from a solution of the nitriles and impurities. A reaction mixture is first formed and to that reaction mixture, 0.1 to 15 wt% of a strong acid (for e.g., a 60% H₂SO₄) is stirred in at room temperature and atmospheric pressure for a time effective for the removal of the amide impurities. Typically, after about 30 minutes, the amide impurity is removed into the lower, dark acid layer, probably as a salt. The remaining nitrile, after decantation and/or filtration, may still be colored but is nearly free of amide. In a second optional color-removing step, at room temperature or at a temperature of up to ∼80°C, color can be removed from the nitrile by stirring the acid-washed nitrile with 0.5-5 wt% of appropriate adsorbent such as clay (bentonite), charcoal and or zeolitic type materials.

Although not limiting the invention to a particular hypothesis, it is believed that the addition of acid to the nitrile containing amide impurities causes the precipitation and/or separation of the amide impurities as acid salts which can be easily removed from the reaction mixture.

The choice of acid to achieve the above is very critical. First, the acid must be strong enough to drive the formation of the amide salts. Water content of the reaction mixture must be controlled so that there is enough to promote insolubility of the acid and its amide salt in the nitrile. Too little water, in the case of a dehydrating acid such as sulfuric, can also lead to "burning" or the promotion of dark colors, especially at elevated temperatures. Hence it is important in the process of the present invention to employ larger amounts of a diluted strong acid rather than smaller amounts of a stronger/concentrated acid. To effect maximum amide removal there should also be an amount of acid in excess of the stoichiometric amount required in order to provide a vehicle for the amide salt and because a portion of the acid is consumed in the course of the various reactions.

Examples of strong acids employable in the context of the present invention include but are not limited to sulfuric acid, hydrochloric acid, hydrobromic acid, perchloric acid, nitric acid, fluorosulfonic acid, methanesulfonic acid, trifluoromethanesulfonic acid, toluenesulfonic acid, phosphoric acid and mixtures thereof. Typically, the acid is diluted in an amount such that its combination with an amount of water that allows an amide salt to remain substantially insoluble in excess aqueous acid. Preferably, 0.1 to 15 wt% of acid is employed. In another embodiment, up to 5 wt% of acid is employed. In still yet another embodiment, 0.5 to 2 wt% acid is employed.

The conditions at which the process of the present invention is carried out are not critical. The present inventors have noticed, however, that the color of the acid-washed nitrile deepens as the sulfuric acid concentration increases or if the temperature is raised. Optimum conditions appear to be obtained with 60-70% H₂SO₄ at about 25°C. A temperature of from about 15° C to about 100°C and reaction time from about 5 minutes to about 4 hours are the contemplated normal operating conditions for a fatty nitriles feedstock having carbon chains of from about 10 to about 22 in length.

After the nitrile is made, 0.5 to 3 wt% of dilute H₂SO₄ (approximately 50-70%), with or without 0.5 to 5% of an optional filter aid, is stirred in at room temperature and atmospheric pressure for a time effective for the removal of the amide impurities. The optional filter aid may be such things as clay, silica or diatomaceous earth. Typically, after about 30 minutes, the amide impurity is removed into the lower, dark acid layer, or is adsorbed onto the filter aid, probably as a salt. The remaining nitrile, after decantation and/or filtration, may still be colored but is substantially free of amide.

In a second optional color-removing step, at room temperature or at a temperature of up to 30 to about 100°C, color can be removed from the nitrile by stirring the acid-washed nitrile with 0.5-5 wt% of a color adsorbent such as clay (bentonite), charcoal and/or zeolitic type materials. This second step can be carried out by means of a slurry of the mineral and the acid-treated nitrile solution or by passing the nitrile over an adsorbent bed. Bentonite clay is an excellent choice for the slurry method and readily disperses in a liquid medium. The slurry may be maintained for the course of the reaction by agitating means such as a stirrer or mixer.

A key step in the process of the present invention is the separation of the nitriles having a reduced content of impurities and improved color from the reaction mixture. This can be accomplished by draining the thick, liquid amide salt layer from the upper purified nitrile layer or by decantation of the nitrile layer. Alternatively, one may add an adsorbent along with the acid treatment and filter out the adsorbent/amide salt/excess acid by means of one or more filter assemblies. Such an assembly is basically a filter medium, such as a filter cloth, where the feedside is exposed to the fluid to be filtered, and the filtrate side is exposed to a lower pressure. Thus, either pressure or vacuum filtration may be employed with any suitable filtering medium.

The following nonlimiting example is presented to illustrate the present invention.

### EXAMPLE

To a 500 g portion of Arneel^{®} TM (untreated tallow nitrile) at ambient temperature under nitrogen was added 5.0 g (1.0 wt%) of 60% sulfuric acid with rapid stirring. After 30 minutes, the dark mixture was allowed to stand for another 30 minutes before the upper nitrile phase was decanted from the dark oily lower layer. The acid-treated nitrile was then stirred with 2.0 wt% of bentonite clay at 80° for 30 minutes before filtration. The changes in color and amide content are shown below.

### Arneel TM after Acid Treatment and Decolorizing

| **Analysis** | **Before treatment** | **After treatment** |
|---|---|---|
| Gardner color | 6.8 | <1 |
| Amides, wt% by IR | 0.19 | <0.02 (undetectable) |

## Claims

1. A process for the removal of long-chain aliphatic amide impurities from a solution of said amides and fatty acid-derived nitriles which comprises washing said solution with an amount of a strong acid effective to remove the amide as a salt in the acid layer, separating said acid layer from said solution leaving an acid-treated fatty acid-derived nitrile substantially free from said amide impurities.

2. The process of claim 1 wherein 0.1 to 15 wt% of said strong acid is employed.

3. The process of claim 1 or 2 wherein said strong acid is selected from the group consisting of sulfuric acid, hydrochloric acid, hydrobromic acid, perchloric acid, nitric acid, fluorosulfonic acid, methanesulfonic arid, trifluoromethanesulfonic acid, toluenesulfonic acid, phosphoric acid and mixtures thereof in combination with an amount of water that allows an amide salt to remain substantially insoluble in excess aqueous acid.

4. The process of any one of the preceding claims wherein said process is conducted at ambient temperature and atmospheric pressure.

5. The process of any one of the preceding claims wherein 0.5 to 5% of filter aid is optionally present.

6. The process of any one of the preceding claims wherein agitation is employed to maximize the contacting of said strong acid and said amide impurity.

7. The process of any one of the preceding claims wherein said strong acid comprises sulfuric acid.

8. The process of claim 7 wherein 50 to 70% sulfuric acid is employed.

9. The process of claim 8 wherein up to 5 wt% of 50 to 70% sulfuric acid solution is employed.

10. The process of any one of the preceding claims which further comprises a decolorization step.

11. The process of claim 10 wherein said decolorization step comprises contacting said acid-treated nitrile with a color-removing adsorbent.

12. The process of claim 11 wherein said adsorbent is selected from the group consisting of clays, activated carbons, alumina, silica gel, zeolites and mixtures thereof.

13. The process of claims 11 or 12 wherein 0.1 to about 5% of said adsorbent is employed.

14. The process of any one of claims 11-13 wherein said adsorbent comprises a bentonite clay, and said reaction mixture is in the form of a slurry of finely divided particles of said clay with said solution.

15. A process for the purification and decolorization of fatty acid-derived nitriles containing long-chain aliphatic amide impurities which comprises washing a solution of said amides and fatty acid-derived nitriles with an amount of a strong acid effective to remove the amide as a salt in the acid layer, separating said acid layer from said solution leaving an acid-treated fatty acid-derived nitrile substantially free from said amide impurities, and thereafter contacting said acid-treated nitrile with an adsorbent in an amount effective for color reduction.

16. The process of claim 15 wherein said strong acid is selected from the group consisting of sulfuric acid, hydrochloric acid, hydrobromic acid, perchloric acid, nitric acid, fluorosulfonic acid, methanesulfonic acid, trifluoromethanesulfonic acid, toluenesulfonic acid, phosphoric acid and mixtures thereof in combination with an amount of water that allows an amide salt to remain substantially insoluble in excess aqueous acid and mixtures thereof.

17. The process of claim 15 or 16 wherein said adsorbent is selected from the group consisting of clays, activated carbons, alumina, silica gel, zeolites and mixtures thereof.

18. The process of any one of claims 15-17 wherein 0.1 to 15 wt% of said strong acid is employed and wherein 0.1 to about 1% of said adsorbent is employed.

19. The process of any one of claims 15-18 wherein said acid is sulfuric acid and said adsorbent comprises a bentonite clay, and said reaction mixture is in the form of a slurry of finely divided particles of said clay with said solution.

20. The process of any one of claims 15-19 wherein said process is conducted at ambient temperature and atmospheric pressure.

## Patentansprüche

1. Verfahren zur Entfernung von langkettigen aliphatischen Amidverunreinigungen aus einer Lösung der Amide und von Fettsäurenitrilen, umfassend das Waschen der Lösung mit einer Menge einer starken Säure, die die Entfernung des Amids als Salz in die Säureschicht bewirkt, das Abtrennen der Säureschicht von der Lösung, wobei ein säurebehandeltes Fettsäurenitril zurückbleibt, das im Wesentlichen frei von den Amidverunreinigungen ist.

2. Verfahren gemäß Anspruch 1, wobei 0,1 bis 15 Gew.-% der starken Säure eingesetzt werden.

3. Verfahren gemäß Anspruch 1 oder 2, wobei die starke Säure aus der Gruppe ausgewählt ist, die aus Schwefelsäure, Chlorwasserstoffsäure, Bromwasserstoffsäure, Perchlorsäure, Salpetersäure, Fluorsulfonsäure, Methansulfonsäure, Trifluormethansulfonsäure, Toluolsulfonsäure, Phosphorsäure und Gemischen davon besteht, in Kombination mit einer Menge Wasser, die es ermöglicht, dass ein Amidsalz in überschüssiger wässriger Säure im Wesentlichen unlöslich bleibt.

4. Verfahren gemäß einem der vorstehenden Ansprüche, wobei das Verfahren bei Umgebungstemperatur und unter Atmosphärendruck durchgeführt wird.

5. Verfahren gemäß einem der vorstehenden Ansprüche, wobei gegebenenfalls 0,5 bis 5% Filterhilfsstoffe vorhanden sind.

6. Verfahren gemäß einem der vorstehenden Ansprüche, wobei gerührt wird, um den Kontakt zwischen der starken Säure und der Amidverunreinigung zu maximieren.

7. Verfahren gemäß einem der vorstehenden Ansprüche, wobei die starke Säure Schwefelsäure umfasst.

8. Verfahren gemäß Anspruch 7, wobei 50- bis 70%-ige Schwefelsäure eingesetzt wird.

9. Verfahren gemäß Anspruch 8, wobei bis zu 5 Gew.-% der 50- bis 70%-igen Schwefelsäurelösung eingesetzt werden.

10. Verfahren gemäß einem der vorstehenden Ansprüche, das weiterhin einen Entfärbungsschritt umfasst.

11. Verfahren gemäß Anspruch 10, wobei der Entfärbungsschritt das In-Kontakt-Bringen des säurebehandelten Nitrils mit einem farbentfernenden Adsorptionsmittel umfasst.

12. Verfahren gemäß Anspruch 11, wobei das Adsorbens aus der Gruppe ausgewählt ist, die aus Tonen, Aktivkohlen, Aluminiumoxid, Silicagel, Zeolithen und Gemischen davon besteht.

13. Verfahren gemäß Anspruch 11 oder 12, wobei 0,1 bis etwa 5% des Adsorbens eingesetzt werden.

14. Verfahren gemäß einem der Ansprüche 11 bis 13, wobei das Adsorbens einen Bentonit-Ton umfasst und das Reaktionsgemisch in Form einer Aufschlämmung von feinteiligen Teilchen des Tons in der Lösung vorliegt.

15. Verfahren zur Reinigung und Entfärbung von Fettsäurenitrilen, die langkettige aliphatische Amidverunreinigungen enthalten, umfassend das Waschen einer Lösung der Amide und der Fettsäurenitrile mit einer Menge einer starken Säure, die die Entfernung des Amids als Salz in die Säureschicht bewirkt, das Abtrennen der Säureschicht von der Lösung, wobei ein säurebehandeltes Fettsäurenitril zurückbleibt, das im Wesentlichen frei von den Amidverunreinigungen ist, und danach das In-Kontakt-Bringen des säurebehandelten Nitrils mit einem Adsorbens in einer Menge, die eine Farbreduktion bewirkt.

16. Verfahren gemäß Anspruch 15, wobei die starke Säure aus der Gruppe ausgewählt ist, die aus Schwefelsäure, Chlorwasserstoffsäure, Bromwasserstoffsäure, Perchlorsäure, Salpetersäure, Fluorsulfonsäure, Methansulfonsäure, Trifluormethansulfonsäure, Toluolsulfonsäure, Phosphorsäure und Gemischen davon in Kombination mit einer Menge Wasser, die es ermöglicht, dass ein Amidsalz in überschüssiger wässriger Säure im Wesentlichen unlöslich bleibt, und Gemischen davon besteht.

17. Verfahren gemäß Anspruch 15 oder 16, wobei das Adsorbens aus der Gruppe ausgewählt ist, die aus Tonen, Aktivkohlen, Aluminiumoxid, Silicagel, Zeolithen und Gemischen davon besteht.

18. Verfahren gemäß einem der Ansprüche 15 bis 17, wobei 0,1 bis 15 Gew.-% der starken Säure eingesetzt werden und wobei 0,1 bis etwa 1% des Adsorbens eingesetzt werden.

19. Verfahren gemäß einem der Ansprüche 15 bis 18, wobei es sich bei der Säure um Schwefelsäure handelt und das Adsorbens einen Bentonit-Ton umfasst und das Reaktionsgemisch in Form einer Aufschlämmung von feinteiligen Teilchen des Tons in der Lösung vorliegt.

20. Verfahren gemäß einem der Ansprüche 15 bis 19, wobei das Verfahren bei Umgebungstemperatur und unter Atmosphärendruck durchgeführt wird.

## Revendications

1. Procédé destiné à l'élimination d'impuretés d'amide aliphatique à longue chaîne d'une solution desdits amides et de nitriles dérivés d'acide gras qui comprend le lavage de ladite solution avec une quantité d'un acide fort efficace pour éliminer les amides sous forme de sel dans la couche acide, la séparation de ladite couche acide de ladite solution en laissant un nitrile dérivé d'acide gras traité à l'acide sensiblement dépourvu desdites impuretés d'amide.

2. Procédé selon la revendication 1, dans lequel 0,1 à 15 % en poids dudit acide fort sont employés.

3. Procédé selon la revendication 1 ou 2, dans lequel ledit acide fort est choisi dans le groupe constitué par l'acide sulfurique, l'acide chlorhydrique, l'acide bromhydrique, l'acide perchlorique, l'acide nitrique, l'acide fluorosulfonique, l'acide méthanesulfonique, l'acide trifluorométhanesulfonique, l'acide toluènesulfonique, l'acide phosphorique et des mélanges de ceux-ci en combinaison avec une quantité d'eau qui permet à un sel d'amide de rester sensiblement insoluble dans un acide aqueux en excès.

4. Procédé selon l'une quelconque des revendications précédentes, dans lequel ledit procédé est réalisé à température ambiante et à pression atmosphérique.

5. Procédé selon l'une quelconque des revendications précédentes, dans lequel 0,5 à 5 % d'adjuvant de filtration sont facultativement présents.

6. Procédé selon l'une quelconque des revendications précédentes, dans lequel une agitation est employée pour maximiser le contact entre ledit acide fort et ladite impureté d'amide.

7. Procédé selon l'une quelconque des revendications précédentes, dans lequel ledit acide fort comprend de l'acide sulfurique.

8. Procédé selon la revendication 7, dans lequel de l'acide sulfurique à 50 à 70 % est employé.

9. Procédé selon la revendication 8, dans lequel jusqu'à 5 % en poids d'une solution d'acide sulfurique à 50 à 70 % sont employés.

10. Procédé selon l'une quelconque des revendications précédentes, qui comprend en outre une étape de décoloration.

11. Procédé selon la revendication 10, dans lequel ladite étape de décoloration comprend la mise en contact dudit nitrile traité à l'acide avec un absorbant décolorant.

12. Procédé selon la revendication 11, dans lequel ledit absorbant est choisi dans le groupe constitué par les argiles, les charbons actifs, l'alumine, le gel de silice, les zéolites et leurs mélanges.

13. Procédé selon la revendication 11 ou 12, dans lequel 0,1 à environ 5 % dudit absorbant sont employés.

14. Procédé selon l'une quelconque des revendications 11 à 13, dans lequel ledit absorbant comprend une argile de bentonite, et ledit mélange de réaction se présente sous la forme d'une boue de particules finement divisées de ladite argile avec ladite solution.

15. Procédé destiné à la purification et à la décoloration de nitriles dérivés d'acides gras contenant des impuretés d'amide aliphatique à longue chaîne qui comprend le lavage d'une solution desdits amides et nitriles dérivés d'acide gras avec une quantité d'un acide fort efficace pour éliminer l'amide sous forme de sel dans la couche acide, la séparation de ladite couche acide de ladite solution en laissant un nitrile dérivé d'acide gras traité à l'acide sensiblement dépourvu desdites impuretés d'amide, puis la mise en contact dudit nitrile traité à l'acide avec un absorbant dans une quantité efficace pour une réduction de couleur.

16. Procédé selon la revendication 15, dans lequel ledit acide fort est choisi dans le groupe constitué par l'acide sulfurique, l'acide chlorhydrique, l'acide bromhydrique, l'acide perchlorique, l'acide nitrique, l'acide fluorosulfonique, l'acide méthanesulfonique, l'acide trifluorométhanesulfonique, l'acide toluènesulfonique, l'acide phosphorique et des mélanges de ceux-ci en combinaison avec une quantité d'eau qui permet à un sel d'amide de rester sensiblement insoluble dans un acide aqueux en excès et des mélanges de celui-ci.

17. Procédé selon la revendication 15 ou 16, dans lequel ledit absorbant est choisi dans le groupe constitué par les argiles, les charbons actifs, l'alumine, le gel de silice, les zéolites et leurs mélanges.

18. Procédé selon l'une quelconque des revendications 15 à 17, dans lequel 0,1 à 15 % en poids dudit acide fort sont employés et dans lequel 0,1 à environ 1 % dudit absorbant est employé.

19. Procédé selon l'une quelconque des revendications 15 à 18, dans lequel ledit acide est l'acide sulfurique et ledit absorbant comprend une argile de bentonite, et ledit mélange de réaction se présente sous la forme d'une boue de particules finement divisées de ladite argile avec ladite solution.

20. Procédé selon l'une quelconque des revendications 15 à 19, dans lequel ledit procédé est réalisé à température ambiante et à pression atmosphérique.
